# EUROPEAN PATENT APPLICATION

(11) **EP 1 498 386 A1**
(43) Date of publication of application: **19.01.2005**
(21) Application number: 03743031.1
(22) Date of filing: 26.02.2003
(51) Int. Cl.: B82B 1/00, B82B 3/00, C07H 15/203, D01F 9/00

(54) **MICROFINE SELF-AGGREGATE**

(30) Priority: 26.02.2002 JP 2002049238
(71) Applicant: Japan Science and Technology Agency, Kawaguchi-shi, Saitama 332-0012 (JP); National Institute of Advanced Industrial Science and Technology, Tokyo 100-8921 (JP)
(72) Inventor: UZAWA, Hirotaka, Tsukuba Center, Tsukuba-shi, Ibaraki 305-8561 (JP); SHIMIZU, Toshimi, Tsukuba Center, Tsukuba-shi, Ibaraki 305-8561 (JP); KAMIYA, Shoko, Tsukuba Center, Tsukuba-shi, Ibaraki 305-8561 (JP); JOHN, George, Tsukuba Center, Tsukuba-shi, Ibaraki 305-8561 (JP)
(74) Representative: Wilson Gunn Gee
(86) International application number: PCT/JP2003/002108
(87) International publication number: WO 2003/072489

(57) **Abstract**

A nanoscale self-aggregate having a unique high order structure was obtained by preparing a nanoscale self-aggregate having an oligosaccharide in the saccharide chain.

The present invention is a nanoscale self-aggregate comprising an O-glycoside type oligolipid having a structure represented by the general formula (1) shown below. 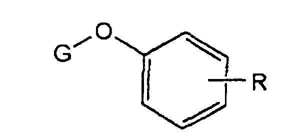 (In the formula, G represents an oligosaccharide radical composed from two to thirty bonded monosaccharides, and R represents a hydrocarbon group containing six to twenty-five carbon atoms.) This nanoscale self-aggregate assumes the shape of micro double helical fiber shaped aggregates, micro helical disk shaped aggregates, nanoscale tubular aggregates and microfiber shaped aggregates.

## Description

### Technical Field

The present invention relates to nanoscale self-aggregates comprising O-glycoside type glycolipids. More specifically, the present invention relates to nanoscale self-aggregates comprising O-glycoside type glycolipids containing oligosaccharides in the saccharide chain.

### Conventional Technology

Cardanyl glycoside is a long chain glucose substituted alkyl phenol derivative traditionally synthesized from purified cardanol starting material separated from natural plant materials. When cardanyl glycoside is heated and dissolved in water and allowed to cool gradually, nanotube shaped aggregates are known to form through hydrogen bonding. [G. John, M. Masuda, Y. Okada, K. Yase and T. Shimizu, Adv. Mat., 13, 715 (2001)]

### Problems for the Invention to Solve

Nanoscale self-aggregates of long chain alkyl phenol derivatives containing saccharide chain substitutions are produced when glucose is the sugar segment. Cardanyl galactoside, a long chain alkyl phenol derivative containing saccharide chain substitution, is similarly prepared when galactose is used in place of glucose. It forms crystals when it is dissolved in hot water and allowed to cool gradually.

Based on this knowledge, an investigation on the morphology of aggregates was conducted in the present invention by preparing a long chain hydrocarbon phenol derivative having saccharide chain substitutions in which there was an oligosaccharide in the saccharide chain.

### Means to Solve the Problems

The present invention provides nanoscale self-aggregates having a unique higher order structure by using a saccharide chain containing an oligosaccharide to add the properties derived from the oligosaccharide to the nanoscale self-aggregates.

That is, the present invention is a nanoscale self-aggregate comprising O-glycoside type oligolipid having a structure represented by the general formula (1) shown below.

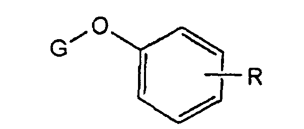

(In the formula, G represents an oligosaccharide radical composed of two to thirty monosaccharides, and R represents a hydrocarbon group containing six to twenty-five carbon atoms.)

### Explanation of Simple Figures

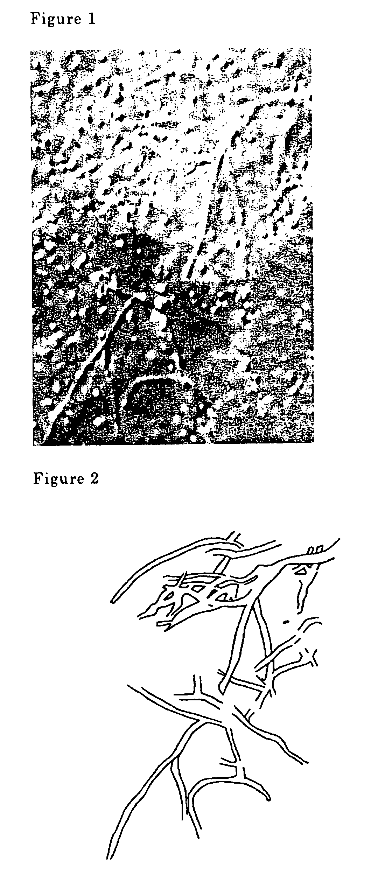

Figure 1 shows an optical microscope image of nanofiber shaped aggregates obtained in Example 1. The photograph size is 175 µm long and 127 µm wide.

Figure 2 is a traced figure of Figure 1.

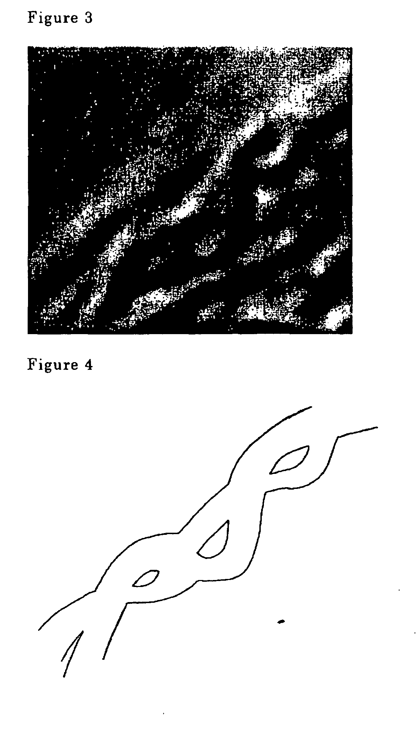

Figure 3 shows an optical microscope image of micro double helical fiber shaped aggregates obtained in Example 2. The photograph scale is 28 µm long and 36 µm wide.

Figure 4 is a traced figure of Figure 3.

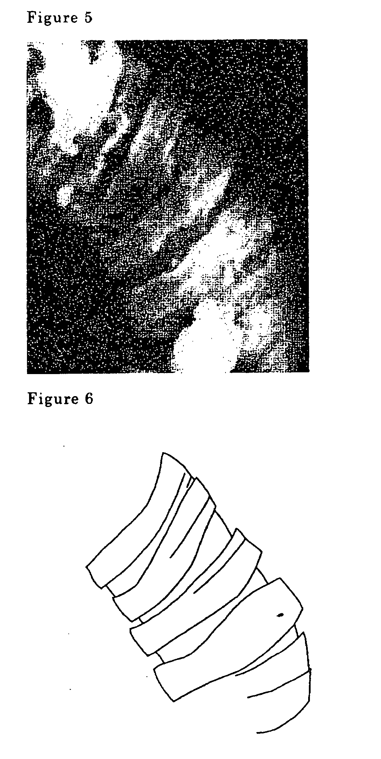

Figure 5 shows an optical microscope image of micro helical disk shaped aggregates obtained in Example 2. The photograph size is 8.3 µm long and 7.1 µm wide.

Figure 6 is a traced figure of Figure 5.

### Embodiment of the Invention

A surface activating organic compound used in the present invention is an o-glycoside type oligolipid represented by the general formula (1) shown below.

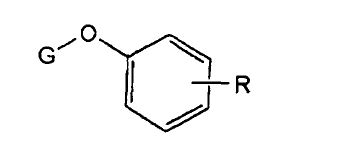

In the present invention, the hydrocarbon group (R) may be located at any one of o-, m- or p-positions to the -O-G group, but the meta (m) position is preferred.

G in the aforementioned general formula (1) is an oligosaccharide chain radical having no reducing terminal hydroxyl group. That is, G is an oligosaccharide radical having a reducing terminal carbon atom involved in an O-glycoside linkage and having a saccharide radical number of 2-30, preferably two to five, more preferably two. Commercially available compounds such as lactose, melibiose, cellobiose and galactosyl-α(1-4) galactose, for example, or oligosaccharides obtained using chemical synthesis or enzymatic synthesis can be mentioned as examples of this type of compound. The reduced terminal anomer position of the glycoside bond may be an α-anomer, β-anomer or a mixture thereof.

The R in the aforementioned general formula is a hydrocarbon group containing from six to 25 carbon atoms, preferably from fourteen to sixteen carbon atoms, more preferably fifteen carbon atoms, and is preferably an aliphatic hydrocarbon comprising a saturated or an unsaturated aliphatic hydrocarbon containing one to five, preferably one to three unsaturated bonds. This hydrocarbon is preferably linear. Dodecyl groups, tridecyl groups, tetradecyl groups, pentadecyl groups, hexadecyl groups, heptadecyl groups and octadecyl groups as well as groups containing mono-enes, dienes, trienes and the like can be mentioned as examples of such a hydrocarbon group. However, a 8-pentadecenyl group, a 8,10-pentadecadienyl group and a 8,10,12-pentadecatrienyl group are preferred from the standpoint of ready raw material availability.

The long chain hydrocarbon phenol derivatives represented by the aforementioned general formula (1) having oligosaccharide substitutions can be manufactured, for example, according to the process shown below.

An oligosaccharide containing hydroxyl groups that are completely protected by acetyl groups is substituted onto a long chain hydrocarbon phenol shown by general formula (2) to obtain a long chain hydrocarbon phenol derivative having oligosaccharide substitution by adding a glycoside linkage to the hydroxyl group and next removing the protective acetyl groups from the saccharide radicals. 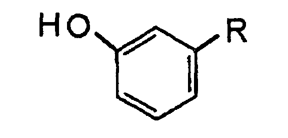 (In the formula, R is as defined previously.)

Commercially available compounds such as lactose octaacetate and cellobiose octaacetate can be used as the oligosaccharides wherein all of the hydroxyl groups are protected by acetyl groups. In addition, commercially available oligosaccharides such as melibiose, 4-O-α-D-galactopyranosyl-D-galactopyranose and the like as well as acetylated oligosaccharides obtained through enzymatic or chemical synthesis may be used.

An example demonstrating the order of acetylation is described as follows. An oligosaccharide is dissolved in dry pyridine, dimethyl aminopyridine is added and the mixture is stirred using a magnetic stirrer at from room temperature to 40°C for an hour to overnight. The reaction progresses quickly when anhydrous acetic acid is added to the reaction system at this point. Pyridine is removed using five to seven co-distillation procedures with a toluene-ethanol mixed solvent, and the residue is dissolved in chloroform. The chloroform solution is washed several times using a saturated aqueous sodium bicarbonate solution and water. The solution is concentrated, purified using a silica gel column and dried through concentration.

A desirable mode for producing an oligosaccharide substituted long chain hydrocarbon phenol derivative of the present invention is explained next.

An oligosaccharide containing two to thirty saccharide radicals and having all of the hydroxyl groups protected by acetyl groups is glycoside bonded to the hydroxyl group of the long chain hydrocarbon phenol represented by the aforementioned general formula (2). In this reaction, trimethylsilyl trifluoromethane sulfonate and boron trifluoride-ethyl ether complex are added as activating agents designed to activate the anomer position on the reducing terminals of oligosaccharide. A desirable ratio of the oligosaccharide, the long chain alkylphenol and the activating agent is 1:1.5:1-1.2, respectively, for the blending and reaction steps. The yield is improved when the types of activating agents and the reaction temperature are changed according to the oligosaccharides and long chain alkyl phenols used. When an oligosaccharide and a long chain alkyl phenol that are difficult to react are being considered, for example, slightly harsher conditions including the use of boron trifluoride-ethyl ether complex and a room temperature reaction temperature are appropriate. However, oligosaccharide chains decompose when conditions are too harsh, and the yield declines. Milder conditions, including the use of trimethylsilyl trifluoromethane sulfonate as the activation agent and a reaction temperature of about 0°C, are desired when relatively reactive oligosaccharides and long chain alkyl phenols are involved. The compound obtained is dissolved in methanol, and a methanol solution of sodium methoxide at room temperature is added to de-acetylate the compound obtained. A long chain hydrocarbon phenol derivative of the aforementioned general formula (1) having oligosaccharide substitutions can be obtained by adding a strongly acidic ion exchange resin to neutralize the solution.

The manufacturing process for a nanoscale self-aggregate of the present invention is not restricted, but it can be obtained by dispersing the aforementioned O-glycoside type oligolipid in water, heating the dispersion using a mantle heater to boil it for about twenty minutes, allowing the reaction mixture to cool naturally to room temperature and allowing it to stand at room temperature until nanotubes are formed. (Japanese Patent Applications 2000-271192 and 2001-363762.) The procedure is explained here in more detail. One milligram of a long chain hydrocarbon phenol derivative of the aforementioned general formula (1) having oligosaccharide substitutions obtained was dispersed in 40 ml of water at 60°C by sonicating. The dispersion was heated to 120°C to dissolve the solids. When the solution was allowed to stand and cool gradually, nanoscale self-aggregates were obtained in the form of an aqueous dispersion. The shapes and sizes of the aggregates obtained were confirmed using an optical microscope and a scanning electron microscope to examine the aggregates present in a solution before drying, obtained from the drops of the solution placed on a substrate and allowed to dry or obtained by freeze drying the solution before placing it on a substrate.

When manufacturing nanoscale self-aggregates, a single type (a single material) of O-glycoside type oligolipid represented by the aforementioned Chemical Formula (1) may be used or a mixture of at least two O-glycoside type oligolipids may also be used (for example, a mixture of O-glycoside type oligolipids comprising natural cardanol, a mixture of four isomers, used in Example 1).

### Effect of the Invention

Nanoscale self-aggregates of the present invention are nanoscale self-aggregates of O-glycoside type oligolipids having high order structures such as, for example, nanofiber shaped aggregates, microfiber shaped aggregates, micro helical fiber shaped aggregates, nanotube shaped aggregates and micro helical disk shaped aggregates, and, in addition, they are nanoscale self-aggregates having added properties of the oligosaccharides used. The nanoscale self-aggregates can be used as pharmaceutical evaluation agents or adsorption agents as well as emulsifiers, stabilizers, dispersion agents, wetting agents and nano- or micro materials in the food chemistry industry, the agricultural and forestry industry, the electronic data industry and the like.

The present invention is demonstrated by the examples shown below, but the examples are not intended to restrict the present invention.

### Production Example 1

Cashew nut oil was vacuum distilled twice at about 400 Pa, and the fraction boiling from 220°C to 235°C was collected to obtain cardanol. To a 100 ml pear shaped flask were added 2.00 g of β-lactose octaacetate (Sigma Co.), 0.89 g of cardanol, 3 g of molecular sieve 4A and 30 ml of dry toluene, and the mixture was agitated using a magnetic stirrer for an hour at room temperature. The reaction system was cooled using an ice bath, and 0.53 ml of trimethylsilyl trifluoromethane sulfonate, measured using a syringe, was added dropwise under a nitrogen atmosphere into the reaction system using a dropping funnel. The reaction system was agitated using a magnetic stirrer for two hours, and 4.1 ml of triethylamine was subsequently added. The reaction system was agitated with a magnetic stirrer for some time to allow the reaction to terminate. The reaction mixture was suction filtered using Cerite while washing it using ethyl acetate, and the filtrate was concentrated. The concentrate was added to a silica gel column (column diameter: 5 cm, column length: 30 cm, silica gel powder volume: 550 ml, development solvent: toluene:ethyl acetate = 2:1, drain: 250 ml), and a fraction collector was used to divide the product into approximately 20 ml increments. Fractions 29 to 53 were collected, and the solvent was removed using a Kugel dryer (60°C, for 30 minutes) to obtain cardanyl-2,3,4,6,2',3',6'-hepta-O-acetyl-β-D-lactoside. The yield was 0.39 g. Rf = 0.45 (developing solvent: toluene:ethyl acetate = 2:1).

Three-hundred milligrams of the cardanyl-2,3,4,6,2',3',6'-hepta-O-acetyl-β-D-lactoside obtained above was next added to a 50 ml pear shaped flask, and 3 ml of methanol and 3 ml of tetrahydrofuran were added to dissolve it. Three drops of a 28% sodium methoxide/methanol solution was added, and the pH of the solution was checked. The pH was nine. The reaction system was agitated using a magnetic stirrer for two hours at room temperature Dowex 50 was added to the reaction system to neutralize it and to terminate the reaction. The reaction system was suction filtered through Cerite while washing with tetrahydrofuran to remove the grains of Dowex 50, and the filtrate was concentrated. The concentrate was added to a silica gel column (column diameter: 5 cm, column length: 30 cm, silica gel powder volume: 450 ml, developing solvent: toluene:ethyl acetate = 2:1, drain: 230 ml), and a fraction collector was used to collect the product at about 10 ml increments. Fractions 41 to 200 were collected and the solvent was removed using a Kugel dryer (60°C, for 30 minutes) to obtain cardanyl (mixed type)-β-D-lactoside. The yield was 184 g. Rf = 0.61 (developing solvent: chloroform:methanol = 10:3).

¹H-NMR (400 MHz, CD₃OD, r.t.): δ7.19-6.82 (m, phenyl group), 5.36 (m, -CH₂CHCH₂-), 4.93 (d, J_{1,2} = 7.2 Hz, H-1) , 4.39 (d, J_{1,2} = 7.6 Hz, H-1'), 3.90-3.13 (m, saccharide chain), 2.79 (m, -CHCHCH₂CHCH-), 2.57 (t, J = 7.6 Hz, -C₆H₄CH₂-), 2.04 (m, -CH₂CH₂CHCHCH₂CH₂-), 1.60-1.29 (m, -CH₂-), 0.90 ppm (m, -CH₃) .

### Example 1

Five milligrams of the cardanyl-β-D-lactoside obtained in Production Example 1 was placed in a 500 ml pear shaped flask, and 200 ml of water was added. A mantle heater was used to reflux the reaction mixture for four hours while agitating it with a magnetic stirrer. The reaction mixture was allowed to stand at room temperature for several days, and a white precipitate was formed. The shape of the precipitate was confirmed using an optical microscope and a transmission type electron microscope, and the nanofibrous aggregates shown in Figures (1) and (2) were observed. The nanofiber shaped aggregates consisted of branched fibers having diameters of about 800 nm.

### Production Example 2

About 5 ml of a cardanol mixture containing saturated type, monoene type, diene type and triene type cardanol separated by distilling cashew nut oil was added to a silica gel column (column diameter: 5 cm, column length: 30 cm, silica gel powder volume: 450 ml, developing solvent: toluene:ethyl acetate = 10:1, drain: 250 ml), and a fraction collector was used to collect the product in about 10 ml increments. Fractions 11 to 23 were collected and the solvent was removed using a Kugel dryer (60°C, for 30 minutes).

The cardanol obtained (584 mg) was added to a silica gel column (column diameter: 3 cm, column length: 30 cm, silica gel powder volume: 200 ml, developing solvent: toluene:ethyl acetate = 9:1, drain: 80 ml), and a fraction collector was used to collect the product in about 7 ml increments. Fractions 40 to 46 were collected and the solvent was removed using a Kugel dryer (60°C, for 30 minutes). The cardanol obtained comprised only a monoene type cardanol. The yield was 143 mg. Rf = 0.63 (developing solvent: hexane:ethyl acetate = 9:1).

¹H-NMR (400 MHz, CD₃OD, r.t.): δ7.16-6.63 (m, phenyl group), 5.34 (m, -CH₂CHCHCH₂-), 4.67 (d, J = 4.4 Hz, -OH), 2.55 (t, J = 7.8 Hz, -C₆H₄CH₂-), 2.02 (m, -CH₂CHCHCH₂-), 1.61-1.26 (m, -CH₂-), 0.88 ppm (t, J = 6.8 Hz, -CH₃).

### Production Example 3

To a 50 ml of a pear shaped flask were added 271 mg of β-lactose octaacetate, 121 mg of the cardanol obtained in Production Example 3 [sic], 0.4 g of molecular sieve 4A and 4 ml of dry toluene, and the mixture was agitated using a magnetic stirrer for an hour at room temperature. The reaction system was cooled using an ice bath, and 72 µl of trimethylsilyl trifluoromethane sulfonate measured using a syringe was added dropwise under an argon atmosphere into the reaction system using a dropping funnel. The reaction system was agitated using a magnetic stirrer for two hours, and 0.6 ml of triethylamine was subsequently added. The reaction system was agitated using a magnetic stirrer for some time to terminate the reaction. The reaction mixture was suction filtered using Cerite while washing using ethyl acetate, and the filtrate was concentrated. The concentrate was added to a silica gel column (column diameter: 2 cm, column length: 30 cm, silica gel powder volume: 80 ml, developing solvent: toluene:ethyl acetate = 2:1, drain: 40 ml) , and a fraction collector was used to collect the product in about 6 ml increments. Fractions 16 to 22 were collected and the solvent was removed using a Kugel dryer (60°C, for 30 minutes) to obtain (monoene type) cardanyl-2,3,4,6,2',3',6'-hepta-O-acetyl-β-D-lactoside. The yield was 54 mg. Rf = 0.39 (developing solvent: toluene:ethyl acetate = 2:1).

Fifty-four milligrams of the (monoene type) cardanyl-2, 3, 4, 6, 2',3', 6'-hepta-O-acetyl-β-D-lactoside obtained above was next added to a 50 ml pear shaped flask, and 1 ml of methanol and 1 ml of tetrahydrofuran were added to dissolve it. One drop of a 28% sodium methoxide/methanol solution was added, and the pH of the solution was checked. The pH was nine. The reaction system was agitated using a magnetic stirrer for two hours at room temperature. Dowex 50 was added to the reaction system to neutralize it and to terminate the reaction. The reaction system was suction filtered through Cerite while washing with tetrahydrofuran to remove the grains of Dowex 50, and the filtrate was concentrated. The concentrate was added to a silica gel column (column diameter: 1 cm, column length: 30 cm, silica gel powder volume: 50 ml, developing solvent: chloroform:methanol = 5:1, drain: 0 ml), and a fraction collector was used to collect the product in about 10 ml increments. Fractions 20 to 45 were collected and the solvent was removed using a Kugel dryer (60°C, for 30 minutes) to obtain (monoene type) cardanyl-β-D-lactoside. The yield was 28 mg. Rf = 0.29 (developing solvent: chloroform:methanol = 10:3).

Elemental analysis: C₃₃H₅₄O₁₁
Calculated: C: 63.24% H: 8.68%
Experimental: C: 68.23% H: 8.86%
¹H-NMR (400 MHz, CD₃OD, r.t.): δ7.19-6.82 (m, phenyl group), 5.34 (m, -CH₂CHCHCH₂-), 4.93 (d, J_{1,2} = 7.6 Hz, H-1), 4.39 (d, J_{1,2} = 7.6 Hz, H-1'), 3.93-3.13 (m, saccharide chain), 2.57 (t, J = 7.8 Hz, -C₆H₄CH₂-), 2.02 (m, -CH₂CHCH₂CH₂-), 1.60-1.28 (m, -CH₂-), 0.90 ppm (t, J = 6.8 Hz, -CH₃).

### Example 2

One milligram of the cardanyl (monoene type)-β-D-lactoside obtained in Production Example 3 was placed in a 200 ml Erlenmeyer flask, and 40 ml of water was added. The flask and the contents were heated for twenty minutes at 120°C in an autoclave after having been sonicated for twenty minutes in a water bath maintained at 60°C. The autoclave remained covered overnight, and the contents and the flask were then removed to room temperature at which point a white precipitate had formed. The shape of the precipitate was confirmed using an optical microscope and a scanning electron microscope, and the examination revealed a mixture of micro double helical fiber shaped aggregates [shown in Figures (3) and (4)], micro helical disk shaped aggregates [shown in Figures (5) and (6)], nanotube shaped aggregates and microfiber shaped aggregates.

The micro double helical fiber shaped aggregates shown in Figures (3) and (4) were aggregates formed from two strands of helical ribbon shaped aggregates about 1 µm wide entangled with each other at mild angles to form a network having a diameter of about 8 µm.

The micro helical disk shaped aggregates shown in Figures (5) and (6) were aggregates formed from helical ribbon shaped aggregates about 1 µm wide wound at acute angles to form a shape almost like a disk with the disks aggregated along central axes to form columnar bodies about 4-5 µm in diameter.

## Claims

1. A nanoscale self-aggregate comprising O-glycoside type oligolipid having a structure represented by the general formula (1) shown below. 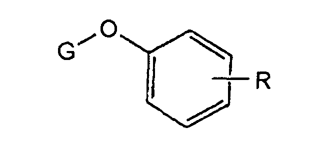 (In the formula, G represents an oligosaccharide radical composed of two to thirty monosaccharides, and R represents a hydrocarbon group containing six to twenty-five carbon atoms.)

2. The nanoscale self-aggregate as in Claim 1 wherein the hydrocarbon group (R) is located in a meta position relative to the -O-G group in the general formula (1).

3. The nanoscale self-aggregate as in Claim 1 or 2 wherein the oligosaccharide is a disaccharide.

4. The nanoscale self-aggregate as in any one of Claims 1-3 wherein the oligosaccharide is lactose.
